# EUROPEAN PATENT APPLICATION

(11) **EP 4 666 932 A1**
(43) Date of publication of application: **24.12.2025**
(21) Application number: 24756577.3
(22) Date of filing: 22.01.2024
(51) Int. Cl.: A61B 1/06, A61B 1/045

(54) **ELECTRONIC ENDOSCOPE SYSTEM**

(30) Priority: 16.02.2023 JP 2023022720
(71) Applicant: HOYA CORPORATION, Shinjuku-ku Tokyo 160-8347 (JP)
(72) Inventor: HAGIWARA Masayuki, Tokyo 160-8347 (JP)
(74) Representative: Lucke, Andreas
(86) International application number: PCT/JP2024/001692
(87) International publication number: WO 2024/171726

(57) **Abstract**

One aspect of the present invention is an electronic endoscope system including an electronic scope including a CMOS image sensor configured to image a biological tissue in a rolling shutter method, a light source device including a light emitting device for generating illumination light for the biological tissue, and a driver signal processing unit. The driver signal processing unit allows the light emitting device to perform a light emission operation on the basis of a signal of a level corresponding to an amplitude of a current allowed to flow through each of the light emitting devices, a current setting signal for setting the current allowed to flow through the light emitting device, and controls the current setting signal in such a manner that a level of the current setting signal rises from zero before a light emission start time of a frame when the CMOS image sensor images.

## Description

### Technical Field

The present invention relates to an electronic endoscope system used in an electronic endoscope including an image sensor configured to image a biological tissue.

### Background Art

In a medical device field, there is known an endoscope system capable of generating an image suitable for diagnosing a lesion hidden in a body cavity, by illuminating a biological tissue in the body cavity and imaging the illuminated biological tissue in the body cavity as an object. Conventionally, a lamp light source such as a xenon lamp or a halogen lamp that emits white light has been used as illumination light; however, recently, a semiconductor light source including a light emitting device such as a light emitting diode (LED) that emits light having a specific wavelength band has been used instead of the lamp light source.

In contrast, a CMOS image sensor is often used as an image sensor for imaging the biological tissue in the body cavity as the object. In a case of using the CMOS image sensor, exposure is sequentially performed while providing a time difference by a rolling shutter method, and image signals are sequentially output (for example, JP 2020-137614 A).

### Summary of Invention

### Technical Problem

In an endoscope system, for example, there is a case where weak pulse light emission is performed when a distance from a distal end portion of an endoscope to an object is short. In such a case, when it is set in such a manner that rising of a pulse of a current setting signal for setting a current of a light emitting device is synchronized with a light emission start time of one frame, a lighting start time of the light emitting device might be delayed from the light emission start time. When the lighting start time of the light emitting device is delayed from the light emission start time, a still image obtained by an image sensor is deteriorated.

Therefore, an object of the present invention is to stabilize a lighting start time of a light emitting device in an electronic endoscope system.

### Solution to Problem

One aspect of the present disclosure is an electronic endoscope system including:
an electronic endoscope including an image sensor configured to image a biological tissue by a rolling shutter method;
a light emitting device that generates illumination light with respect to the biological tissue;
a light emission driving unit that allows the light emitting device to perform a light emission operation on the basis of a signal of a level corresponding to an amplitude of a current allowed to flow through the light emitting device, a current setting signal for setting the current allowed to flow through the light emitting device; and
a control unit that controls the current setting signal in such a manner that a level of the current setting signal rises from zero before a light emission start time of a frame when the image sensor images.

The control unit may control the current setting signal so that a value of the current allowed to flow through the light emitting device before the light emission start time becomes 10 times or more a minimum value of a current that can be output by the light emitting device.

A first period in which the level of the current setting signal is set to a first level from a light emission start time of a first frame may be set. In this case, the level of the current setting signal is set to be negative in a predetermined period from an end time of the first period to a light emission start time of a second frame subsequent to the first frame.

A start time of the predetermined period preferably is an end time of the first period or immediately after the end time.

The control unit may set
a first period in which the level of the current setting signal is set to a first level from a light emission start time of a first frame, and
a second period in which the level of the current setting signal is set to a second level smaller than the first level from an end time of the first period.

In this case, the level of the current setting signal is set to be negative in a predetermined period from an end time of the second period to a light emission start time of a second frame subsequent to the first frame.

A start time of the predetermined period preferably is an end time of the second period or immediately after the end time.

The electronic endoscope system may include a plurality of light emitting devices, and a plurality of light emission driving units that allows a corresponding light emitting device among the plurality of light emitting devices to perform a light emission operation.

In this case, when light generated by each light emitting device by the light emission start time is set as first light, the control unit preferably adjusts the current setting signal for each light emitting device so that a light amount ratio of a light amount of the first light to a predetermined reference light amount becomes the same among the plurality of light emitting devices or falls within a predetermined allowable variation value before the biological tissue is imaged.

The electronic endoscope system may include a light amount acquisition unit that acquires a light amount of the first light and the reference light amount on the basis of a first image of an object obtained when the object serving as a reference is irradiated with the first light and a second image of the object obtained when the object is irradiated with the first light and second light, which is light generated by each light emitting device after the light emission start time before the biological tissue is imaged. In this case, the control unit calculates the light amount ratio with respect to each light emitting device on the basis of the light amount acquired by the light amount acquisition unit.

The control unit may adjust a time at which the level of the current setting signal rises from zero so that a peak level of the current setting signal for generating the first light becomes a predetermined value.

### Advantageous Effects of Invention

According to the electronic endoscope system described above, the lighting start time of the light emitting device can be stabilized.

### Brief Description of Drawings

Fig. 1 is a block diagram illustrating an example of a configuration of an electronic endoscope system of one embodiment.
Fig. 2 is a timing chart for describing a global exposure operation by a CMOS image sensor.
Fig. 3 is a block diagram illustrating a configuration of a light source device included in the system in Fig. 1.
Fig. 4 is a diagram for describing a shift between a current setting signal and an LED lighting start timing as a problem of the conventional technology.
Fig. 5 is a timing chart illustrating a current setting signal and an LED lighting state in the electronic endoscope system of one embodiment.
Fig. 6 is a diagram for describing a shift between the current setting signal and an LED lighting start timing.
Fig. 7 is a timing chart illustrating a current setting signal and an LED lighting state in the electronic endoscope system of one embodiment.
Fig. 8 illustrates an example of a waveform indicating light amounts of a plurality of LEDs in one frame, illustrating a state in which a preliminary light emission period varies.
Fig. 9 is a block diagram of a substantial part of an electronic endoscope in the electronic endoscope system of one embodiment.
Fig. 10 is a flowchart illustrating calibration with respect to preliminary light emission in the electronic endoscope system of one embodiment.
Fig. 11 is a diagram for describing a light amount in a preliminary light emission period and a reference light emission period.
Fig. 12 illustrates an example of a waveform indicating the light amounts of a plurality of LEDs in one frame, illustrating a state after the calibration.
Fig. 13 is a block diagram illustrating an example of a configuration of an electronic endoscope system in which a light source device is embedded in a processer.

### Description of Embodiments

An electronic endoscope system according to the present embodiment will be hereinafter described in detail with reference to the drawings.

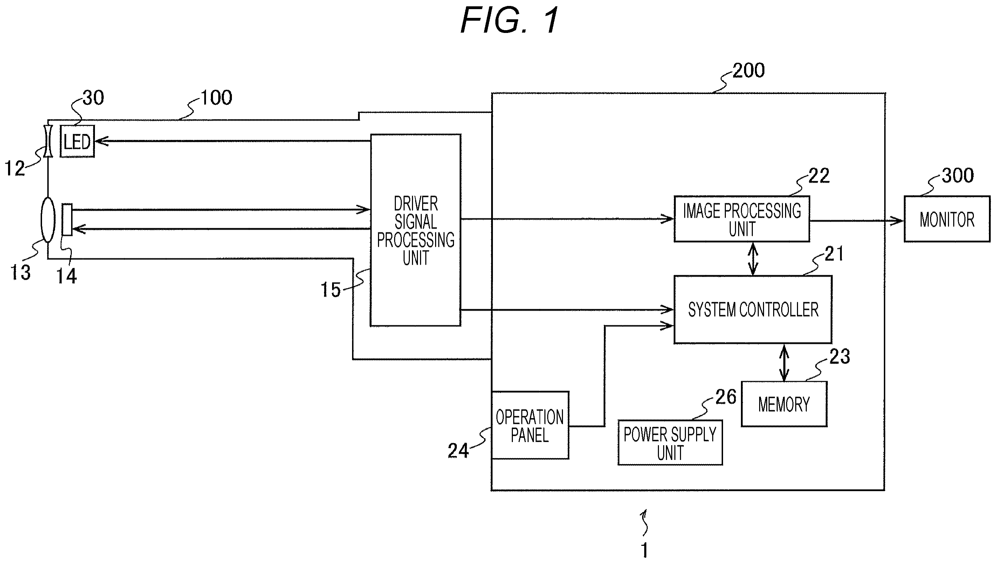

Fig. 1 is a block diagram illustrating an example of a configuration of an electronic endoscope system 1 of the present embodiment. As illustrated in Fig. 1, the electronic endoscope system 1 is a system specialized for medical use, and includes an electronic scope (endoscope) 100, a processor for an electronic endoscope 200 (hereinafter, simply referred to as a "processor 200"), and a monitor 300.

The processor 200 includes a system controller 21. The system controller 21 executes various programs stored in a memory 23 and integrally controls an entire electronic endoscope system 1. The system controller 21 is connected to an operation panel 24. The system controller 21 changes each operation of the electronic endoscope system 1 and a parameter for each operation in accordance with an instruction from an operator input to the operation panel 24. The system controller 21 supplies a clock pulse serving as a reference of timing of an operation of each unit in the electronic endoscope system 1 to each unit in the system.

A distal end portion of the electronic scope 100 is provided with a light source device 30. The light source device 30 includes a light emitting device (LED) that emits illumination light for illuminating an object such as a biological tissue in a body cavity. The illumination light is white light or pseudo white light. The white light is light having a flat spectral intensity distribution in a visible light band, and the pseudo white light is light obtained by mixing light of a plurality of wavelength bands having a non-flat spectral intensity distribution.

The object is irradiated with the illumination light of the light source device 30 through a light distribution lens 12. Return light from the object illuminated with the illumination light from the light distribution lens 12 forms an optical image on a light receiving surface of a CMOS image sensor 14 via an objective lens 13.

The complementary metal oxide semiconductor (CMOS) image sensor 14 (an example of an image sensor) is an image sensor having a Bayer pixel array. The CMOS image sensor 14 reads an optical image formed by each pixel on the light receiving surface as charges corresponding to a light amount to generate and output imaging data. Note that, the CMOS image sensor 14 may be replaced with a CCD image sensor and other types of imaging devices. The CMOS image sensor 14 may include a complementary color filter.

The electronic scope 100 includes a driver signal processing unit 15 in a connection unit to the processor 200.

The driver signal processing unit 15 performs processing of driving and controlling the CMOS image sensor 14 and the light source device 30, and acquires the imaging data from the CMOS image sensor 14.

In one embodiment, the driver signal processing unit 15 supplies a synchronization signal (for example, a vertical synchronization signal) of one frame to the CMOS image sensor 14, acquires the imaging data of the object from the CMOS image sensor 14 in units of frames, and transmits the same to an image processing unit 22 of the processor 200. A frame cycle is, for example, 1/30 seconds or 1/60 seconds. Note that, the present invention is not limited to a case where the driver signal processing unit 15 supplies the synchronization signal of one frame. Some CMOS image sensors transmit the imaging data on the basis of the synchronization signal created by the CMOS image sensor itself. In this case, it is not necessary for the driver signal processing unit 15 to supply the synchronization signal of one frame to the CMOS image sensor.

The driver signal processing unit 15 transmits, to the light source device 30, a current setting signal for controlling light emission timing of the LED and an LED current allowed to flow through the LED. The current setting signal and a configuration of the light source device 30 will be described later.

In the electronic scope 100 illustrated in Fig. 1, a rolling shutter method is used as an exposure/output method for exposing the light receiving surface of the CMOS image sensor 14 and outputting as an image. The rolling shutter method is a method in which the light receiving surface of the image sensor is divided into a plurality of pixel regions in units of pixels, and exposure is sequentially performed while providing a time difference for each pixel region, the method in which accumulated charges are sequentially reset for each pixel region, and then accumulation of charges by exposure is started to output (read) accumulated charges to be an image signal. The pixel region is, for example, one line or a plurality of consecutive lines.

In the electronic endoscope system 1, illumination light is turned on in a pulsed manner at regular intervals to illuminate the biological tissue. At that time, a global exposure period is provided in such a manner that an exposure period in all effective pixel regions includes a lighting period of the illumination light. By providing the global exposure period, deterioration in image quality due to distortion specific to the rolling shutter method (rolling shutter distortion) is prevented.

After buffering the imaging data output from the driver signal processing unit 15 and performing predetermined image processing on the imaging data, the image processing unit 22 of the processor 200 generates a video format signal and outputs the same to the monitor 300. Examples of the image processing include demosaic processing, matrix calculation, edge enhancement processing and the like.

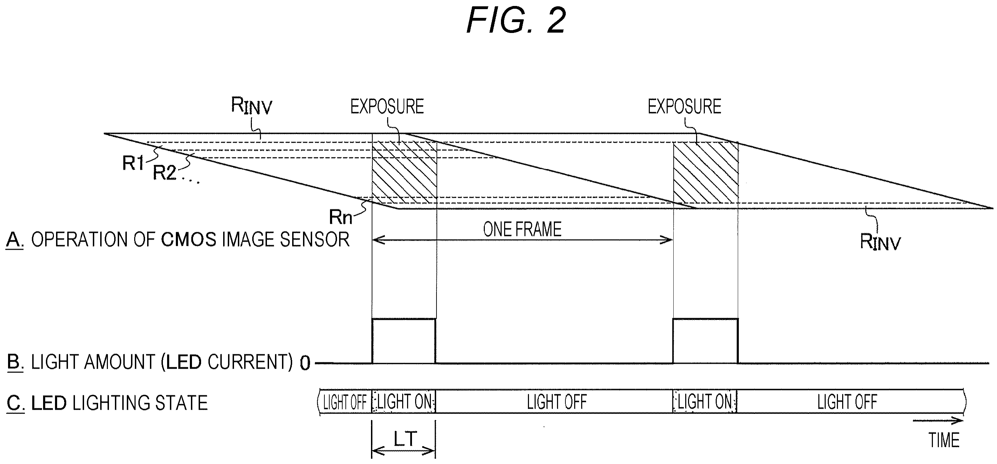

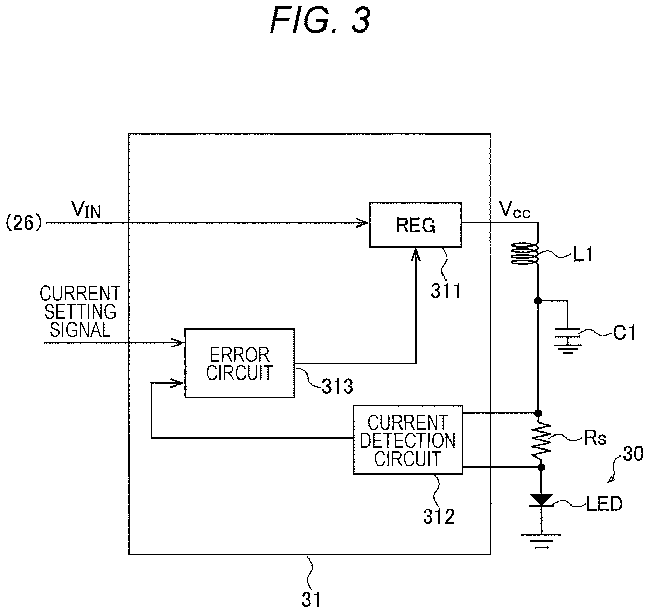

A timing chart in Fig. 2 illustrates an operation of the CMOS image sensor 14 in the electronic endoscope system 1. As illustrated in B and C of Fig. 2, in the processor 200, a current (LED current) corresponding to a required light amount is allowed to flow to a light emitting device (LED) included in the light source device 30, whereby a pulsed lighting period LT is set. Note that, either an analog dimming method or a pulse width modulation (PWM) dimming method is generally applied to the LED. In an example illustrated in Fig. 3, the analog dimming method is illustrated, but the present invention is not limited thereto, and the PWM dimming method may be applied.

As illustrated in A of Fig. 2, in the CMOS image sensor 14, exposure in one frame is started while providing a time difference in the order of pixel regions R1, R2,..., and Rn in the effective pixel regions excluding an ineffective pixel region R_{INV}. Here, the exposure period in all the pixel regions R1, R2,..., and Rn is configured to include the lighting period LT of the LED, and a shaded portion in the drawing can be said to be a substantial exposure period.

Next, a lighting operation of the LED will be described together with a schematic configuration of a light emission driving unit 31 with reference to a block diagram in Fig. 3.

The driver signal processing unit 15 includes the light emission driving unit 31 for driving the LED in the light source device 30.

In one embodiment, the light emission driving unit 31 is a circuit that drives the LED to emit light by the analog dimming method, and includes a voltage regulator 311, a current detection circuit 312, and an error circuit 313. In the light emission driving unit 31, the light emission timing of the LED and the LED current allowed to flow through the LED are controlled by the current setting signal generated by a CPU not illustrated in the electronic scope 100. Note that, the present invention is not limited to a case where the CPU generates the current setting signal, and an application specific integrated circuit (ASIC), a field-programmable gate array (FPGA) and the like may generate the current setting signal.

As illustrated in Fig. 3, a voltage Vcc for obtaining the LED current is generated on the basis of an input voltage V_{IN} supplied from the power supply unit 26 to the voltage regulator 311. One end of an inductor L1 is connected to an output terminal (terminal that outputs the voltage Vcc) of the voltage regulator 311. A sense resistor Rs and the LED are connected in parallel with a capacitor C1 to the other end of the inductor L1.

The sense resistor Rs is provided to detect a current flowing from the voltage regulator 311 to the LED, and both ends thereof are connected to the current detection circuit 312. The current detection circuit 312 detects a current that constantly flows from a voltage across the sense resistor Rs to the LED. The light emission driving unit 31 performs feedback control on the basis of the current flowing through the sense resistor Rs to stabilize the LED current. That is, the error circuit 313 obtains an error between a target current value corresponding to a level of the current setting signal supplied from the driver signal processing unit 15 and an LED current value obtained from the current flowing through the sense resistor Rs. The voltage regulator 311 adjusts the voltage Vcc in such a manner that the error becomes zero. As a result, the light emission driving unit 31 controls so that the LED current having an amplitude corresponding to the current setting signal can be obtained.

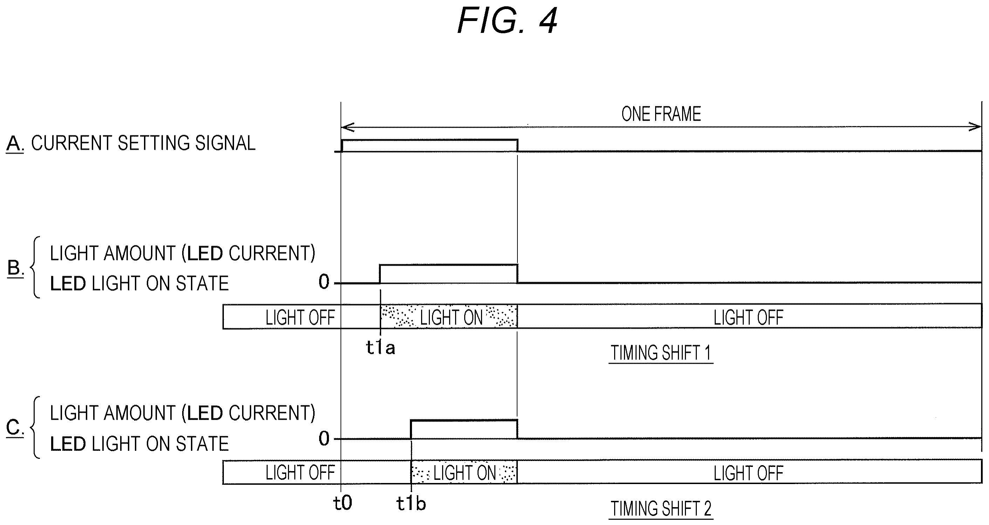

Here, as illustrated in Fig. 2, in a case where the current setting signal is controlled in such a manner that the level of the current setting signal rises from zero in accordance with a start time of the global exposure period of one frame, a time at which the lighting of the LED actually starts might vary before and after. Fig. 4 is a timing chart illustrating two comparative examples (timing shift 1 and timing shift 2) in a case where the lighting start time of the LED is shifted from a rise time of the current setting signal. Here, the "lighting start time" is the time at which lighting of the LED is actually started.

A of Fig. 4 assumes a case where the current setting signal is set to a predetermined level corresponding to the target current value of the LED within one frame period in accordance with the lighting period LT in Fig. 2. Note that, in the following description, a "light emission start time" is a time serving as a reference when the LED is allowed to emit light in units of one frame, and corresponds to a start time to of the global exposure period in Fig. 4.

However, the time when the LED current rises and the lighting of the LED actually starts (lighting start time) is delayed from the light emission start time to, and a delay time varies. In the example of B of Fig. 4, the lighting start time is t1a, and in the example of C of Fig. 4, the lighting start time is t1b later than t1a. The lighting start time is delayed from the light emission start time, and the delay time varies in this manner, due to variation in elements in the circuit of the voltage regulator 311 and ambient temperature. The delay time ((t1a - to) and (tib - to) in Fig. 4) varies depending on the magnitude of the LED current (that is, the magnitude of the level of the current setting signal). Particularly, in a case where weak light is emitted from a light off state (that is, the LED current is changed from zero to a minute value), a response delay of the voltage regulator 311 is likely to occur, and the delay time tends to be long.

Therefore, the processor 200 of the present embodiment is configured to stabilize the lighting start time regardless of the magnitude of the LED current by providing a preliminary light emission period. The preliminary light emission period is a period in which the level of the current setting signal is made larger than zero before the start time to of the global exposure period (that is, the light emission start time). This is performed by the driver signal processing unit 15 controlling timing (particularly, a time when rising from zero) of the current setting signal supplied to the light emission driving unit 31, and the level thereof (particularly, a peak level). In the following description, the light emission of the LED in the preliminary light emission period might be referred to as "preliminary light emission".

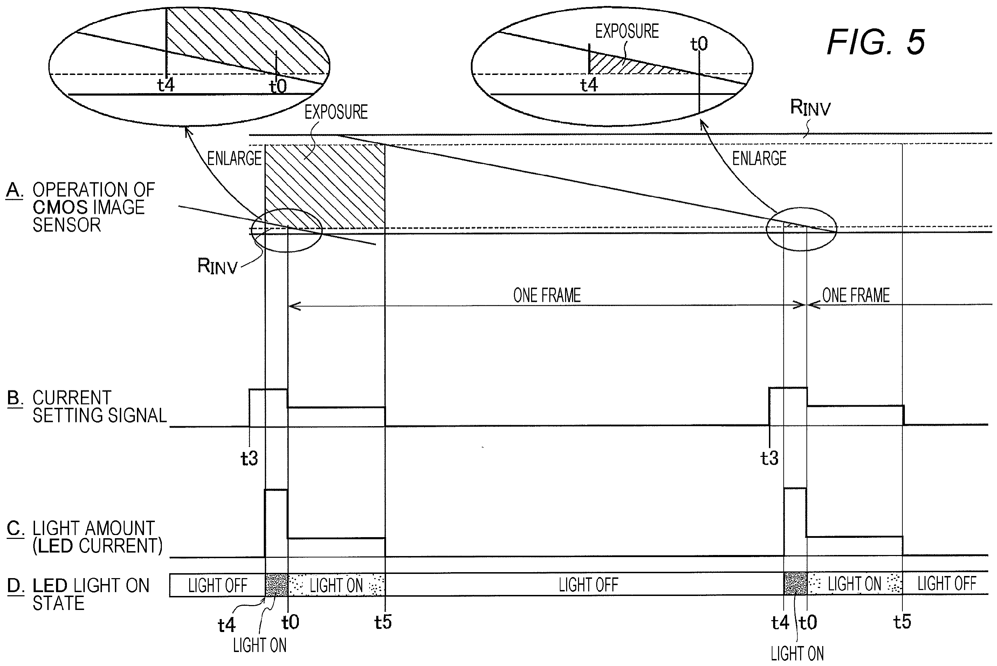

Fig. 5 is a timing chart illustrating the operation of the CMOS image sensor, the current setting signal, the light amount of the LED, and the LED lighting state in the electronic endoscope system 1 of the present embodiment. As illustrated in Fig. 5, the current setting signal is controlled so as to rise from zero at a time t3 before the start time to of the global exposure period, whereby lighting is started at a time t4 before the time to.

The level of the current setting signal in the preliminary light emission period is preferably increased to such an extent that the level is hardly affected by the variation in elements in the circuit of the voltage regulator 311 and the ambient temperature. Since the preliminary light emission period is not the global exposure period, the rolling shutter distortion occurs in a region of several lines at the bottom of a screen (refer to an enlarged view in Fig. 5); however, if the preliminary light emission period is made as short as possible so that the region in which the rolling shutter distortion occurs is about two lines at the bottom of the screen at most, it is not noticeable as an image. Therefore, it is preferable that the preliminary light emission period be as short as possible, and the level of the current setting signal in the preliminary light emission period be increased to such an extent that the rolling shutter distortion is not noticeable.

By providing the preliminary light emission period, the LED current having the magnitude corresponding to the level of the current setting signal can be allowed to flow stably from the start time to of the global exposure period. When light intensity of the preliminary light emission is relatively large, an effect of stabilizing the lighting start time is exhibited.

In the global exposure period, for example, the current setting signal is set in such a manner that the level of the LED current becomes 1 to 200 mA (variable). In the preliminary light emission period, for example, the current setting signal is set in such a manner that the level of the LED current is fixed at 100 mA. Preferably, the system controller 21 controls the current setting signal in such a manner that the value of the current allowed to flow through the LED before the start time to of the global exposure period is 10 times or more a minimum value of the current that can be output by the LED. The reason is as follows.

In the voltage regulator 311, the voltage Vcc to be supplied to the LED is generated by an operation of an internal switching element; the switching operation stops when the current setting signal becomes zero. Thereafter, the current setting signal rises from zero with the start of the preliminary light emission period, and the switching operation in the voltage regulator 3 11 is restarted, but the switching operation is unstable immediately after the restart due to the ambient temperature of the voltage regulator 311 and characteristic variation of the voltage regulator 311. When a period in which the switching operation is unstable overlaps with the preliminary light emission period, in a case where the level of the current setting signal in the preliminary light emission period is small, the error between the LED current value obtained from the current flowing through the sense resistor Rs and the target current value (value corresponding to the level of the current setting signal) is small, so that the voltage Vcc generated by the voltage regulator 311 does not respond rapidly (that is, an increasing rate of the voltage Vcc is low). As a result, the period in which the switching operation is unstable becomes long.

In contrast, in a case where the level of the current setting signal in the preliminary light emission period is set to a value corresponding to the target current value that is 10 times or more the minimum value of the current that can be output by the LED, even if the switching operation is unstable immediately after the restart, the error between the LED current value obtained from the current flowing through the sense resistor Rs and the target current value is sufficiently large, so that the voltage Vcc generated by the voltage regulator 311 rapidly responds. Therefore, since a state in which the switching operation is unstable immediately transits to a state in which the switching is stably performed, the lighting start time is further stabilized.

Note that, in a case where the light amount of the LED only in the global exposure period is not sufficient as the light amount of the LED required in one frame, the LED is additionally turned on in a period other than the global exposure period (an example of a first period) (referred to as an "additional light emission period"; an example of a second period) in some cases. The light amount of the LED in the additional light emission period is auxiliary to the light amount of the LED in the global exposure period, and is thus smaller than the light amount of the LED in the global exposure period. That is, a level of the current setting signal (an example of a second level) in the additional light emission period is smaller than the level of the current setting signal (an example of a first level) in the global exposure period.

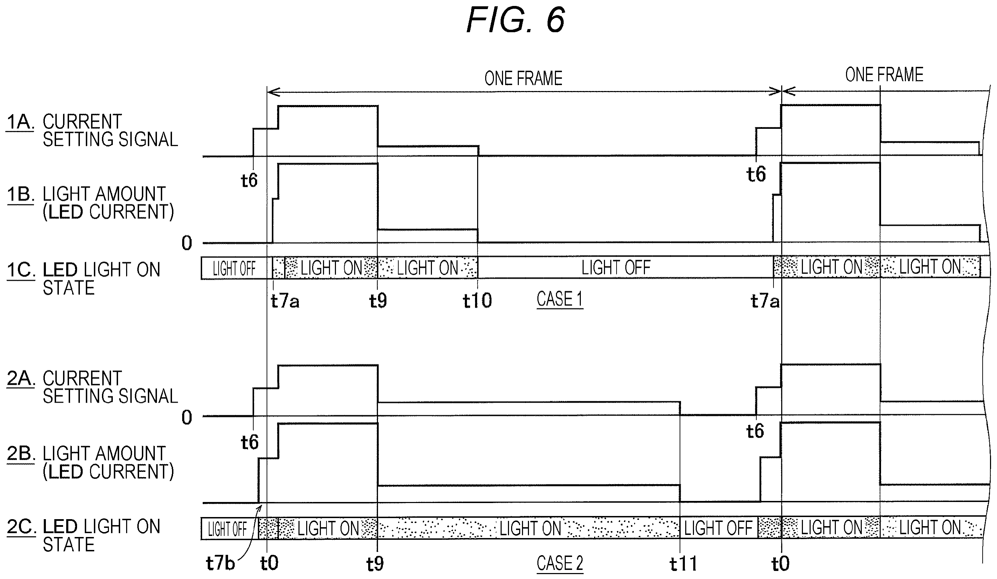

In a case where such preliminary light emission period is provided, the lighting start time of the LED might vary. The variation in the lighting start time is illustrated in Fig. 6. The level of the current setting signal in the additional light emission period is set to be smaller than the level of the current setting signal in the global exposure period. In the additional light emission period, for example, the current setting signal is set in such a manner that the level of the LED current becomes 1 to 30 mA (variable).

Fig. 6 illustrates a timing chart illustrating the operation of the CMOS image sensor 14, the current setting signal, the light amount (LED current), and the LED lighting state for each of cases 1 and 2. In Fig. 6, the global exposure period is from the time to to a time t9 in both of the cases 1 and 2.

In the case 1, the additional light emission period is relatively short between the time t9 and a time t10. At that time, in a case where the preliminary light emission period is provided by raising the current setting signal from zero at a time t6 before the start time to of the global exposure period, the lighting start time is a time t7a after the time to.

In contrast, in the case 2, the additional light emission period is relatively long between the time t9 and a time t11. At that time, also in a case where the preliminary light emission period is provided by raising the current setting signal from zero at the time t6 before the start time to of the global exposure period as in the case 1, the lighting start time is a time t7b after the time to.

The lighting start time varies between the cases 1 and 2 in Fig. 6 due to a difference in length of the additional light emission period. This is because of the following reason.

Even when the additional light emission period in a certain frame ends (that is, even if the current setting signal is set to zero), since there is an LC component (inductor L1 and capacitor C1 in Fig. 3) connected to the LED, the LED current actually continues to flow, and the LED current gradually approaches zero. Due to this transient phenomenon, the LED voltage at the start of the preliminary light emission period in a next frame varies depending on the length of the additional light emission period, and as a result, the lighting start time varies.

Therefore, in one embodiment, in order to eliminate an influence of the transient phenomenon, the level of the current setting signal is set to be negative in a predetermined period from an end time of the additional light emission period of a certain frame to a start time of the preliminary light emission period of a next frame. As a result, regardless of the length of the additional light emission period, the LED current at the start time of the preliminary light emission period of the next frame can be made zero, and the lighting start time of the LED can be stabilized.

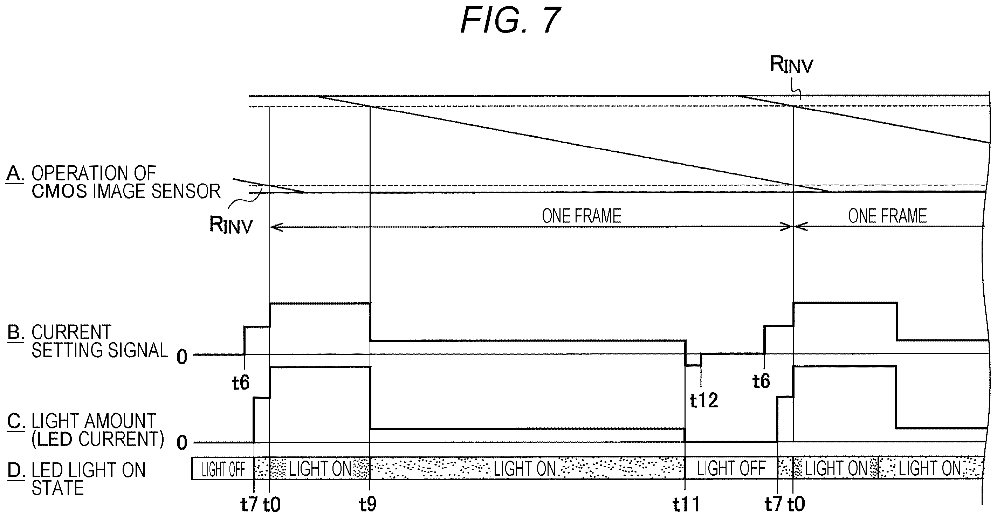

Fig. 7 illustrates a timing chart illustrating the operation of the CMOS image sensor 14, the current setting signal, the light amount (LED current), and the LED lighting state in a case where the predetermined period in which the level of the current setting signal is set to be negative is provided. In the example illustrated in Fig. 7, the current setting signal is set to be negative within the predetermined period within a period from an end time t11 of the additional light emission period of a certain frame to the start time t6 of the preliminary light emission period of a next frame.

Preferably, as illustrated in Fig. 7, the start time of the predetermined period in which the level of the current setting signal is set to be negative is the end time t11 of the additional light emission period or immediately after the same (a period from the time t11 to a time t12). That is, since it takes time to discharge from the LC component connected to the LED after setting the level of the current setting signal to be negative, in order to reliably turn off the LED at the start time of the preliminary light emission period, it is preferable to set the level of the current setting signal to be negative as soon as possible after the additional light emission period ends.

The time "immediately after the end time t11" is not limited, but is, for example, any time within 1 ms from the end time t11.

Similarly, also in a case where the additional light emission period is not provided, the level of the current setting signal is preferably set to be negative in a predetermined period from the end time of the global exposure period of a certain frame to the start time of the preliminary light emission period of a next frame. This is because, depending on the length and/or light amount of the global exposure period, the LED voltage in the global exposure period of a certain frame might not sufficiently drop by the start time of the preliminary light emission period corresponding to the next frame.

Preferably, the start time of the predetermined period in which the level of the current setting signal is set to be negative is the end time of the global exposure period or immediately after the same.

The time "immediately after the end time of the global exposure period" is not limited, but is, for example, any time within 1 ms from the end time of the global exposure period.

As described above, according to the electronic endoscope system 1 of the present embodiment, the driver signal processing unit 15 controls the current setting signal supplied to the light emission driving unit 31 so that the level of the current setting signal rises from zero before the light emission start time of the frame when the CMOS image sensor 14 images, and thus, it is possible to stabilize the lighting start time of each LED.

Considering that the LED voltage does not immediately drop even if the level of the current setting signal is set to zero, it is preferable to set the level of the current setting signal to be negative in the predetermined period from the end time of the global exposure period of a certain frame, or from the end time of the additional light emission period in a case where there is the additional light emission period, to the start time of the preliminary light emission period of the next frame. Accordingly, the lighting start time of the LED can be further stabilized.

In one embodiment, LEDs of a plurality of channels are provided in the light source device 30, and the plurality of LEDs is arranged in parallel on an emission surface of the irradiation light.

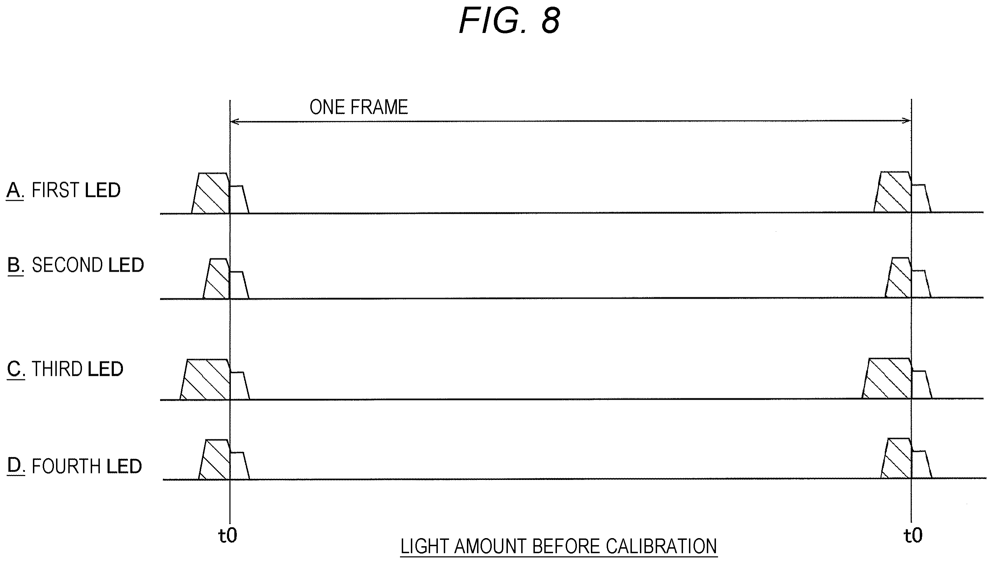

In this case, the LED of each channel generates white light or pseudo white light for irradiating the biological tissue, but the preliminary light emission period of each LED and the light amount in the preliminary light emission period may vary (hereinafter, collectively referred to as "variation in preliminary light emission") due to variation and aging in the plurality of LEDs and/or variation in drive circuit that drives each LED. Fig. 8 is an example of a waveform indicating the light amounts of the plurality of LEDs in one frame, and illustrates a state in which the preliminary light emission period varies. The example illustrated in Fig. 8 is an example in which four-channel LEDs are provided, and the light amount of preliminary light emission (light emission before the light emission start time to) in a first LED and a third LED is larger than that in a second LED and a fourth LED.

In a case where the variation in preliminary light emission is large, an LED having a preliminary light emission period longer than necessary is included among the plurality of LEDs, and a light amount of the preliminary light emission (an integral value of light intensity with respect to time) of the LED is too large, there is a problem that the light amount cannot be appropriately adjusted when the distal end of the electronic scope 100 is brought close to the object. That is, when the distal end of the electronic scope 100 is brought close to the object, it is necessary to reduce the light amount of the LED; however, in a case where the light amount of the preliminary light emission is too large, the preliminary light emission is dominant with respect to an entire light amount, and even if the light amount after the light emission start time is reduced, the entire light amount cannot be sufficiently reduced.

In a case where there is large variation in preliminary light emission among the plurality of LEDs, there also is a problem that brightness on the screen varies depending on positions of the plurality of LEDs arranged in parallel on the emission surface of the irradiation light.

The above-described variation in preliminary light emission and the problem associated therewith can be eliminated or reduced by executing calibration described below before the operator diagnoses the biological tissue (that is, before the biological tissue is imaged).

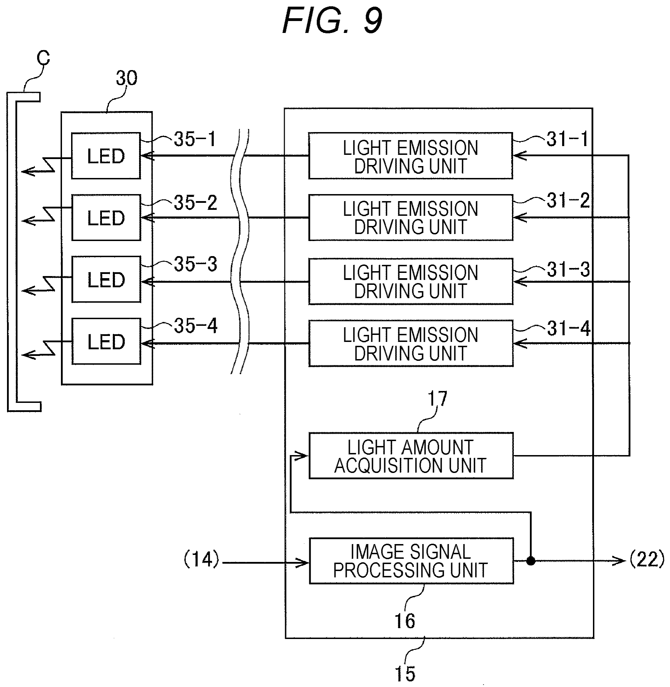

Fig. 9 illustrates a block diagram of a substantial part of the electronic scope 100 in a case where LEDs of a plurality of channels are provided in the light source device 30.

With reference to Fig. 9, the light source device 30 includes a plurality of (for example, four) LEDs 35-1 to 35-4. The driver signal processing unit 15 includes light emission driving units 31-1 to 31-4 that allows the four LEDs 35-1 to 35-4 to perform a light emission operation, respectively. Each of the light emission driving units 31-1 to 31-4 has the same configuration as that of the light emission driving unit 31 illustrated in Fig. 3. In the following description, when the item common to the light emission driving units 31-1 to 31-4 is described, this is denoted as the "light emission driving unit 31".

The driver signal processing unit 15 further includes an image signal processing unit 16 and a light amount acquisition unit 17. The image signal processing unit 16 performs noise removal processing and the like on the image signal acquired by the CMOS image sensor 14. The light amount acquisition unit 17 performs processing of acquiring the light amount on the basis of the image signal subjected to the signal processing by the image signal processing unit 16. The light amount is calculated on the basis of, for example, luminance of the image signal.

When executing the calibration of the preliminary light emission, each of the light emission driving units 31-1 to 31-4 adjusts the preliminary light emission on the basis of the luminance acquired by the light amount acquisition unit 17.

Note that, although not illustrated, the electronic scope 100 includes a CPU, and the CPU executes a program to perform calibration processing of the preliminary light emission. Note that, the electronic scope 100 may include an ASIC, an FPGA and the like that performs calibration processing of the preliminary light emission, instead of the CPU.

In one embodiment, as illustrated in Fig. 9, a white balance adjusting instrument C is installed at the distal end of the electronic scope 100, and white balance is adjusted, or the calibration of the preliminary light emission is executed before or after the white balance is adjusted. The white balance adjusting instrument C is an example of an object serving as a reference and is a white tubular instrument. Note that, the instrument used when performing the calibration of the preliminary light emission is not limited to the white instrument as long as the light amount acquired on the basis of the same irradiation light does not vary, and may be an instrument serving as an object serving as a reference of a color other than white.

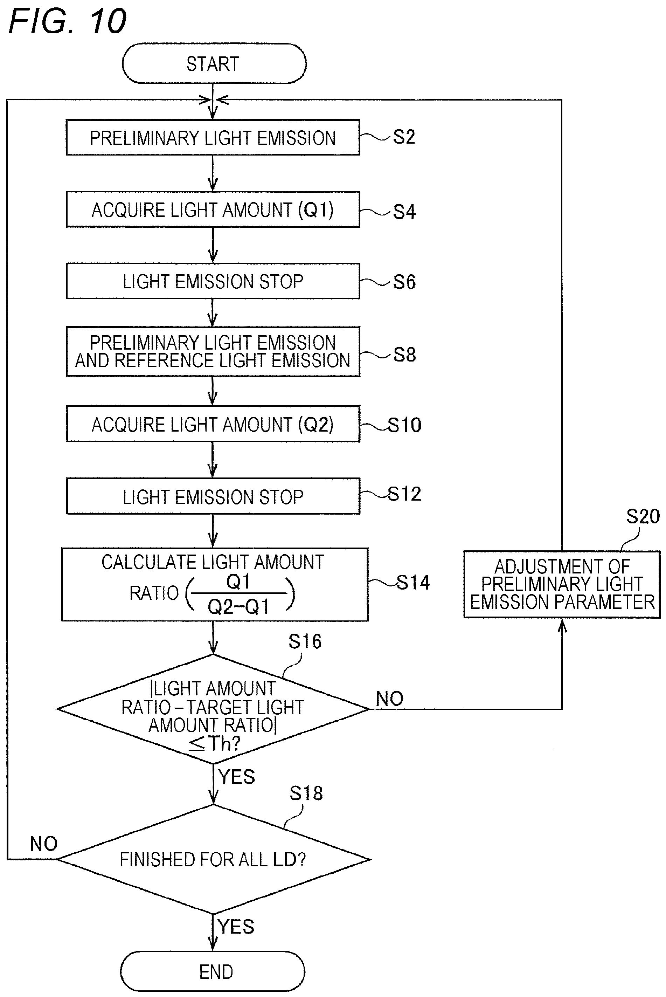

Next, the calibration of the preliminary light emission by a plurality of LEDs will be described with reference to a flowchart in Fig. 10.

The calibration processing in Fig. 10 is executed before the operator diagnoses the biological tissue. With reference to Fig. 10, the calibration processing is performed by sequentially executing processes at steps S2 to S16 for each of the plurality of LEDs (step S18).

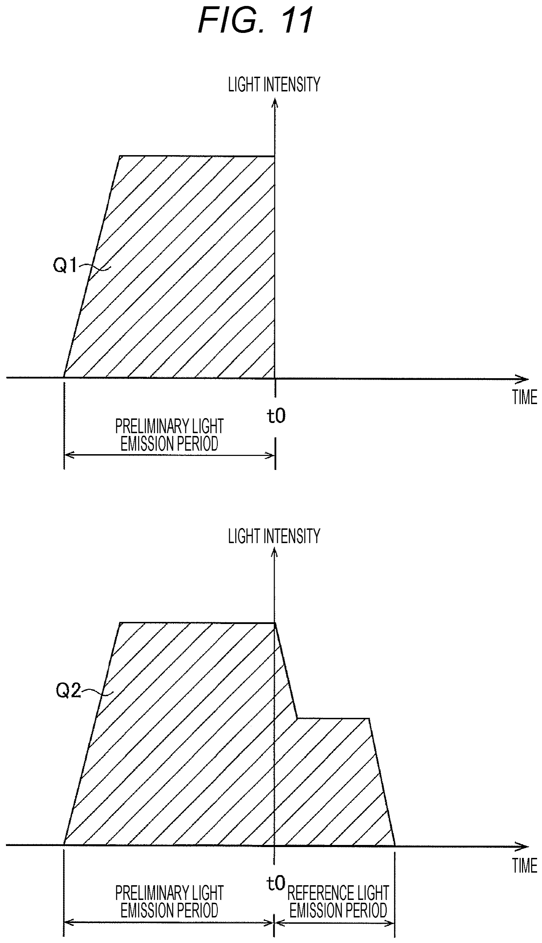

First, the electronic scope 100 controls the light emission driving unit 31 corresponding to the LED to be processed so as to perform the preliminary light emission (an example of first light) before the light emission start time (step S2). The electronic scope 100 acquires a light amount Q1 of the preliminary light emission on the basis of the image signal (an example of the first image) obtained by irradiating the white balance adjusting instrument C with the preliminary light emission (step S4), and then stops the light emission (step S6). As illustrated in Fig. 11, the light amount Q1 of the preliminary light emission is the light amount (time integral value of the light intensity) acquired in the preliminary light emission period before the light emission start time to. Note that, when the light amount Q1 of the preliminary light emission is acquired, it is preferable to suppress the variation in the light amount by averaging the light amounts acquired a plurality of times.

Then, the electronic scope 100 controls the light emission driving unit 31 corresponding to the LED to be processed so as to perform the preliminary light emission and reference light emission (an example of second light) after the light emission start time (step S8). Here, the "reference light emission" means the light emission of a predetermined light amount performed after the light emission start time. The reference light emission is performed by, for example, allowing a predetermined current to flow through the LED to be processed for a predetermined time after the light emission start time.

The electronic scope 100 acquires a light amount Q2, which is the sum of the preliminary light emission and the reference light emission, on the basis of the image signal (an example of the second image) obtained by irradiating the white balance adjusting instrument C with the preliminary light emission and reference light emission (step S10), and then stops the light emission (step S12). As illustrated in Fig. 11, the light amount Q2 is a total light amount (time integral value of the light intensity) including the light amount Q1 acquired in the preliminary light emission period before the light emission start time to and the light amount acquired after the light emission start time to. Note that, when the light amount Q2 is acquired, it is preferable to suppress the variation in the light amount by averaging the light amounts acquired a plurality of times.

Next, the electronic scope 100 calculates a light amount ratio of the light amount Q1 of the preliminary light emission to a reference light amount (Q2-Q1) (Q1/(Q2 - Q1); light amount ratio of preliminary light emission) on the basis of the light amounts Q1 and Q2 acquired at steps S4 and S10, respectively (step S14). The electronic scope 100 determines whether an absolute value of a difference between the light amount ratio calculated at step S14 and the known target light amount ratio is equal to or smaller than a predetermined threshold Th (step S16). In a case where the absolute value of the difference is not equal to or smaller than the threshold Th (step S16: NO), a preliminary light emission parameter is adjusted, and the procedure returns to step S2. The preliminary light emission parameter is a parameter for varying the light amount Q1 of the preliminary light emission, and is a time when the current setting signal rises from zero in the preliminary light emission period and/or the peak level of the current setting signal. The peak level of the current setting signal corresponds to a peak value of the light intensity of the preliminary light emission.

In one embodiment, the electronic scope 100 repeatedly executes steps S2 to S16 while changing the preliminary light emission parameter stepwise until the absolute value of the difference becomes equal to or smaller than a predetermined threshold Th.

In a case where the absolute value of the difference is equal to or smaller than the predetermined threshold Th (step S16: YES), the electronic scope 100 returns to step S2 to execute processing on the next processing target LED as long as the processing is not completed for all the LEDs (step S18: NO).

By executing the above calibration processing, for all the LEDs, the light amount ratio calculated at step S14 is adjusted to be the same among the plurality of LEDs or to fall within a predetermined allowable variation value. The allowable variation value is not limited, but is, for example, within ±10% of the target light amount ratio.

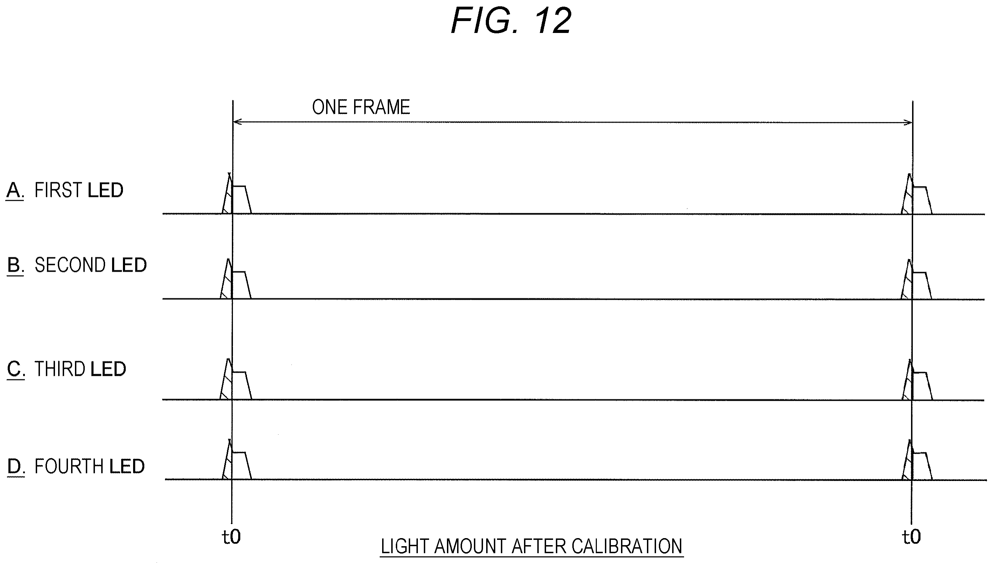

Fig. 12 is an example of a waveform indicating the light amounts of the plurality of LEDs in one frame, and illustrates a state after the calibration of the preliminary light emission. In Fig. 12, as compared with the state illustrated in Fig. 8 (state before the calibration), the light amount of the preliminary light emission is the same among all the LEDs, or falls within a predetermined allowable variation value.

In Fig. 10, it is adjusted such that the light amount ratio of the preliminary light emission to the plurality of LEDs is the same as the known target light amount ratio or the difference therebetween falls within a predetermined allowable variation value, but the present invention is not limited thereto. For example, the light amount ratio calculated for the LED to be processed first may be set as the target light amount ratio, and the preliminary light emission parameter of the LED to be processed second and thereafter may be adjusted such that the light amount ratio calculated for the LED to be processed second and thereafter is the same as the target light amount ratio or falls within a predetermined allowable variation value.

When the light amount of the preliminary light emission is adjusted by adjusting the preliminary light emission parameter at step S20 of Fig. 10, it is preferable to preferentially adjust the time when the current setting signal rises from zero rather than the peak level of the current setting signal. That is, when the light amount of the preliminary light emission is adjusted, it is preferable to preferentially adjust the preliminary light emission period rather than the light intensity of the preliminary light emission. Since the light intensity of the preliminary light emission has high contribution to stabilization of the lighting start time of the LED, it is preferable to adjust the length of the preliminary light emission period after fixing the peak value of the light intensity of the preliminary light emission (that is, the peak level of the current setting signal) to a value large to some extent.

In one embodiment, a system is disclosed in which a light source device is embedded in a processor.

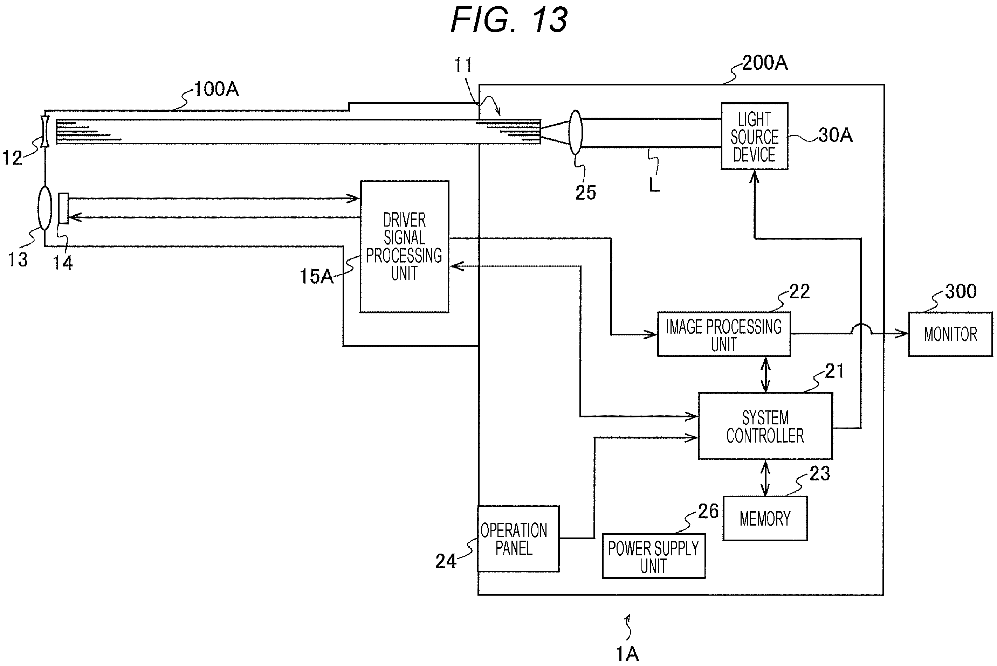

Fig. 13 is a block diagram illustrating an example of a configuration of an electronic endoscope system in which the light source device is embedded in the processer. The system illustrated in Fig. 13 includes an electronic scope 100A and a processor 200A. The processor 200A incorporates a light source device 30A including an LED that emits white light or pseudo white light as the illumination light L. The illumination light L emitted from the light source device 30A is condensed onto an incident end face of a light carrying bundle (LCB) 11 by a condenser lens 25, and enters the LCB 11 of the electronic scope 100A. The illumination light L entering the LCB 11 propagates through the LCB 11. The illumination light L propagating through the LCB 11 is emitted from an exit end face of the LCB 11 arranged at a distal end of the electronic scope 100A, and irradiates a biological tissue (object) via a light distribution lens 12.

A driver signal processing unit 15A of the electronic scope 100A is different from the driver signal processing unit 15 (Fig. 1) in that a light emission drive unit is not provided. The driver signal processing unit 15A notifies a system controller 21 of information regarding a timing of one frame. In the system illustrated in Fig. 13, the light source device 30A includes a light emission driving unit 31 corresponding to an LED, and a light amount of the LED is adjusted by a current setting signal supplied from the system controller 21.

In one embodiment, in a case of detecting the light amount of the LED at the time of calibration of the preliminary light emission in the system illustrated in Fig. 13, the system controller 21 may detect the light amount of the LED on the basis of the image signal acquired by the image processing unit 22.

In one embodiment, the light source device 30A illustrated in Fig. 13 may include a photodetector that detects the light intensity or the light amount of the illumination light emitted from the LED. When detecting the light amount of the LED at the time of calibration of the preliminary light emission, the system controller 21 acquires the light intensity or the light amount detected by the light detector.

The electronic endoscope system according to the present invention has been described in detail above, but the electronic endoscope system according to the present invention is not limited to the above-described embodiment, and may of course be modified or altered in various manners in a range not deviating from the gist of the present invention.

The present invention relates to a patent application of Japanese Patent Application No. 2023-22720 filed with the Japan Patent Office on February 16, 2023, the entire contents of which are incorporated herein by reference.

## Claims

1. An electronic endoscope system comprising:
an electronic endoscope including an image sensor configured to image a biological tissue by a rolling shutter method;
a light emitting device that generates illumination light with respect to the biological tissue;
a light emission driving unit that allows the light emitting device to perform a light emission operation on the basis of a signal of a level corresponding to an amplitude of a current allowed to flow through the light emitting device, a current setting signal for setting the current allowed to flow through the light emitting device; and
a control unit that controls the current setting signal in such a manner that a level of the current setting signal rises from zero before a light emission start time of a frame when the image sensor images.

2. The electronic endoscope system according to claim 1, wherein
the control unit controls the current setting signal so that a value of the current allowed to flow through the light emitting device before the light emission start time becomes 10 times or more a minimum value of a current that can be output by the light emitting device.

3. The electronic endoscope system according to claim 1 or 2, wherein
a first period in which the level of the current setting signal is set to a first level from a light emission start time of a first frame is set, and
the level of the current setting signal is set to be negative in a predetermined period from an end time of the first period to a light emission start time of a second frame subsequent to the first frame.

4. The electronic endoscope system according to claim 3, wherein
a start time of the predetermined period is an end time of the first period or immediately after the end time.

5. The electronic endoscope system according to claim 1 or 2, wherein
the control unit sets
a first period in which the level of the current setting signal is set to a first level from a light emission start time of a first frame, and
a second period in which the level of the current setting signal is set to a second level smaller than the first level from an end time of the first period, and
sets the level of the current setting signal to be negative in a predetermined period from an end time of the second period to a light emission start time of a second frame subsequent to the first frame.

6. The electronic endoscope system according to claim 5, wherein
a start time of the predetermined period is an end time of the second period or immediately after the end time.

7. The electronic endoscope system according to claim 1 or 2, comprising:
a plurality of the light emitting devices; and
a plurality of the light emission driving units that allows a corresponding light emitting device among the plurality of the light emitting devices to perform a light emission operation, wherein
when light generated by each light emitting device by the light emission start time is set as first light, the control unit adjusts the current setting signal for each light emitting device so that a light amount ratio of a light amount of the first light to a predetermined reference light amount becomes the same among the plurality of light emitting devices or falls within a predetermined allowable variation value before the biological tissue is imaged.

8. The electronic endoscope system according to claim 7, comprising:
a light amount acquisition unit that acquires a light amount of the first light and the reference light amount on the basis of a first image of an object obtained when the object serving as a reference is irradiated with the first light and a second image of the object obtained when the object is irradiated with the first light and second light, which is light generated by each light emitting device after the light emission start time before the biological tissue is imaged, wherein
the control unit calculates the light amount ratio with respect to each light emitting device on the basis of the light amount acquired by the light amount acquisition unit.

9. The electronic endoscope system according to claim 7, wherein
the control unit adjusts a time at which the level of the current setting signal rises from zero so that a peak level of the current setting signal for generating the first light becomes a predetermined value.
